# EUROPEAN PATENT SPECIFICATION

(11) **EP 3 906 839 B1**
(45) Date of publication and mention of the grant of the patent: **03.05.2023**
(21) Application number: 18945350.9
(22) Date of filing: 31.12.2018
(51) Int. Cl.: A61B 5/22, A61B 5/00, A61B 5/026, A61B 5/1455, A61B 5/11

(54) **METHOD AND SYSTEM FOR MONITORING BODY TISSUE**
VERFAHREN UND SYSTEM ZUR ÜBERWACHUNG VON KÖRPERGEWEBE
PROCÉDÉ ET SYSTÈME DESTINÉS À SURVEILLER LE TISSU CORPOREL

(43) Date of publication of application: 10.11.2021
(73) Proprietor: OBE Lab., Inc., Seoul 06211 (KR)
(72) Inventor: CHOI, Jong Kwan, Seoul 06094 (KR); HAN, Il Taek, Yongin-si, Gyeonggi-do 16845 (KR); LEE, Ji Seon, Incheon 21357 (KR)
(74) Representative: RGTH
(86) International application number: PCT/KR2018/016986
(87) International publication number: WO 2020/141617

(56) References cited:
- WO-A1-2017/132404
- JP-A- 2014 208 268
- JP-A- 2017 176 266
- KR-A- 20150 100 092
- KR-B1- 100 340 240
- KR-B1- 101 801 473
- US-A1- 2013 102 907
- US-A1- 2016 022 223
- US-A1- 2017 273 576

## Description

### TECHNICAL FIELD

The present disclosure relates to a method, a system, and a non-transitory computer-readable recording medium for monitoring a body tissue.

### BACKGROUND

Conventionally, a technique for directly measuring bioactivity occurring in the body tissue like muscle based on a biosignal, such as ElectroCardioGraphy (ECG), ElectroEncephalography (EEG), has been widely used. However, since such a biosignal has a significant amount of noise which are generated when a subject to be measured moves, it is not suitable for use as a signal for constantly monitoring the bioactivity of the body tissue while the subject is in daily life.

Near-infrared spectroscopy (NIRS) that has been used recently is a method of indirectly analyzing a bioactivity occurring in a body tissue (e.g., brain, muscle, or other body tissues) of a person by measuring the degree of attenuation of near-infrared light (due to scattering and absorption of an optical signal by oxidized or non-oxidized hemoglobin) which varies with hemodynamics changes (e.g., changes in concentrations of oxidized and non-oxidized hemoglobin) due to the bioactivity of the body tissue. As an example, a case of monitoring hemodynamics changes due to the neural activity occurring in the brain is described in detail. Near-infrared light having a wavelength range of about 630 nm to 1300 nm may be transmitted through a skull of the person to the depth of about 1 cm to 3 cm from the skull. By irradiating such near-infrared light to a brain of the head of the person and detecting the near-infrared light reflected or scattered therefrom, or transmitted therethrough, it is possible to monitor hemodynamics changes (e.g., a change in a concentration of blood oxygen, i.e. concentration of oxidized hemoglobin) occurring in the cerebral cortex of the person. According to the near-infrared spectroscopy, the neural activity occurring in a human brain (particularly, a cortex) may be quantified by arranging near-infrared light irradiation modules (i.e., light emission units) and near-infrared light detection modules (i.e., light reception units) at predetermined intervals in various sites of the head of the person, and analyzing signals related to hemodynamics (e.g., optical density (OD) signals based on the near-infrared spectroscopy) specified from optical signals generated by the light emission units and detected by the light reception units.

For example, WO 2017/132404 A1 relates to the use of near-infrared spectroscopy for the sensing of characteristics of muscle tissue.

In US 2013/102907 A1 is a technology disclosed that separates and removes influence of a surface component such as a scalp blood flow component to be mixed in a signal component in a biological photometric device using visible light or near-infrared light.

US 2016/022223 A1 describes systems and methods relating to monitoring tissue at a plurality of depths.

US 2017/273576 A1 relates to a living-body information measuring device comprising a measurement unit that measures living-body information at plural depths in the living body by using the reflected light beams that are successively received by the light-receiving element.

Since the near-infrared spectroscopy uses signals related to the near-infrared optical density with very little noise caused by the motion of the subject, the near-infrared spectroscopy has the advantage of being utilized as a technique which is capable of constantly monitoring the bioactivity of the body tissue during the daily life of the subject.

In this connection, the inventors present a technique for estimating the bioactivity occurring in the body tissue on an area and depth basis by measuring hemodynamics of the body tissue (such as muscle) on the area and depth basis, and furthermore, and evaluating the functional characteristics of the body tissue of the subject.

### SUMMARY

One object of the present disclosure is to solve all the problems described above.

Another object of the present disclosure is to: determine, with reference to a position of an m-th light emitting unit among a plurality of light emitting units configured to generate light signals toward the body tissue to be monitored, a position of an n-th light reception unit among a plurality of light reception units configured to detect the light signals from the body tissue, and a distance between the m-th light emitting unit and the n-th light reception unit, an are an a depth of the body tissue which corresponds to a pair of the m-th light emitting unit and the n-th light reception unit; measure hemodynamics of the body tissue at the area and the depth corresponding to the pair of the m-th light emitting unit and the n-th light reception unit based on the optical signal generated by the m-th light emitting unit and detected by the n-th light reception unit; estimate an activity of the body tissue on an area basis and a depth basis based on the hemodynamics at at least one of the area and the depth of the body tissue; and evaluate functional characteristics of the body tissue of the subject to be measured.

The representative configurations of the present disclosure to achieve the above objects will be described below.

According to one aspect of the present disclosure, a method of monitoring a body tissue includes: determining, with reference to a position of an m-th light emitting unit among a plurality of light emitting units configured to generate optical signals toward the body tissue to be monitored, a position of an n-th light reception unit among a plurality of light reception units configured to detect the optical signals from the body tissue, and a distance between the m-th light emitting unit and the n-th light reception unit, an area and a depth of the body tissue which corresponds to a pair of the m-th light emitting unit and the n-th light reception unit; measuring hemodynamics of the body tissue at the area and the depth corresponding to the pair of the m-th light emitting unit and the n-th light reception unit based on the optical signal generated by the m-th light emitting unit and detected by the n-th light reception unit; and estimating an activity of the body tissue on an area basis and a depth basis based on the hemodynamics at at least one of the area and the depth of the body tissue.

According to another aspect of the present disclosure, a system for monitoring a body tissue includes: a near-infrared spectroscopy (NIRS) measurement management unit configured to determine, with reference to a position of an m-th light emitting unit among a plurality of light emitting units configured to generate optical signals toward the body tissue to be monitored, a position of an n-th light reception unit among a plurality of light reception units configured to detect the optical signals from the body tissue, and a distance between the m-th light emitting unit and the n-th light reception unit, an area and a depth of the body tissue which corresponds to the pair of the m-th light emitting unit and the n-th light reception unit, measure hemodynamics of the body tissue at the area and the depth corresponding to the pair of the m-th light emitting unit and the n-th light reception unit based on the optical signal generated by the m-th light emitting unit and detected by the n-th light reception unit, and estimate an activity of the body tissue on an area basis and a depth basis based on the hemodynamics at at least one of the area and the depth of the body tissue; and a monitoring management unit configured to provide information on an activity of the body tissue on an area basis and a depth basis.

In addition, there are further provided other methods and systems to implement the present disclosure, as well as non-transitory computer-readable recording medium recording having stored thereon computer programs for executing the methods.

According to the present disclosure, since the hemodynamics of the body tissue (e.g., muscle) can be measured on an area basis and a depth basis, it is possible to provide effects of estimating a bioactivity occurring in the body tissue on an area basis and a depth basis, and evaluating functional characteristics (e.g., exercise efficiency of the muscle) of the body tissue of the subject.

Further, according to the present disclosure, since the activity of the muscle of the subject can be expressed on an area basis and a depth basis, it is possible to achieve an effect of providing a muscular strengthening training, a muscular endurance training, a rehabilitation training, or the like customized for the subject.

Furthermore, according to the present disclosure, it is possible to provide an effect of evaluating the exercise efficiency of the muscle of the subject by referring to information on the muscle activity of the subject and information on the exercise quantity of the subject. The present invention is limited by the scope of the subject-matter of the appended claims.

### BRIEF DESCRIPTION OF THE DRAWINGS

FIG. 1 illustratively shows an external configuration of a monitoring device according to one embodiment of the present disclosure.
FIG. 2 illustratively shows an internal configuration of the monitoring system according to one embodiment of the present disclosure.
FIG. 3 illustratively shows an arrangement of a plurality of light emitting units and a plurality of light reception units according to one embodiment of the present disclosure.
FIG. 4 illustratively shows how a depth of a body tissue to be measured varies with a distance between a pair of a light emitting unit and a light reception unit, which defines a measurement channel according to one embodiment of the present disclosure.
FIG. 5 illustratively shows how a depth of a body tissue to be measured varies with a distance between a pair of a light emitting unit and a light reception unit, which defines a measurement channel according to one embodiment of the present disclosure.
FIG. 6A illustratively shows a configuration in which the activity of a muscle being exercised is measured for each region based on an optical signal measured from the muscle being exercised according to one embodiment of the present disclosure.
FIG. 6B illustratively shows a configuration in which the activity of the muscle being exercised is measured for each region based on an optical signal measured from the muscle being exercised according to one embodiment of the present disclosure.
FIG. 7 illustratively shows a configuration in which the activity of the muscle being exercised is measured for each depth based on the optical signal measured from the muscle according to one embodiment of the present disclosure.

### DETAILED DESCRIPTION

In the following detailed description of the present disclosure, references are made to the accompanying drawings that show, by way of illustration, specific embodiments in which the present disclosure may be practiced. These embodiments are described in sufficient detail to enable those skilled in the art to practice the present disclosure. It is to be understood that the various embodiments of the present disclosure, although different from each other, are not necessarily mutually exclusive. For example, specific shapes, structures and characteristics described herein may be implemented as modified from one embodiment to another. Furthermore, it shall be understood that the positions or arrangements of individual elements within each of the disclosed embodiments may also be modified. Therefore, the following detailed description is not to be taken in a limiting sense, and the scope of the present disclosure, if properly described, is limited only by the appended claims. In the drawings, like reference numerals refer to the same or similar functions throughout the several views.

Hereinafter, preferred embodiments of the present disclosure will be described in detail with reference to the accompanying drawings to enable those skilled in the art to easily implement the present disclosure.

Herein, hemodynamics to be monitored by the monitoring device and the monitoring system may include blood composition (e.g., oxyhemoglobin concentration, deoxyhemoglobin concentration, blood oxygen saturation, etc.), the amount of blood flow, the amount of blood volume, and hemodynamics by muscle depth.

### Configuration of Monitoring System

Hereinafter, a monitoring device 100 and a monitoring system 200 which perform major functions to implement the present disclosure will be described.

The monitoring device 100 according to one embodiment of the present disclosure may be worn on (or attached to) a body portion (e.g., head, leg, or the like) of a subject (i.e., a person to be measured), and may function to monitor the activity (e.g., neuronal brain activity, hemodynamic changes in muscle, or the like) occurring in a body tissue located at the corresponding body portion of the subject by measuring a predetermined signal from the subject, and by processing or analyzing the measured signal as described below.

Specifically, the monitoring device 100 according to one embodiment of the present disclosure may include a plurality of light emitting units (or sources) configured to irradiate near-infrared light to a body tissue located in a body portion of a subject, and a plurality of light reception units (or detectors) configured to detect the near-infrared light which are reflected or scattered from, or transmitted through the body tissue (more specifically, the venous blood therein). For example, signals measured by the plurality of light emitting units and the plurality of light reception units included in the monitoring device 100 according to one embodiment of the present disclosure may be optical density (OD) signals based on the near-infrared spectroscopy.

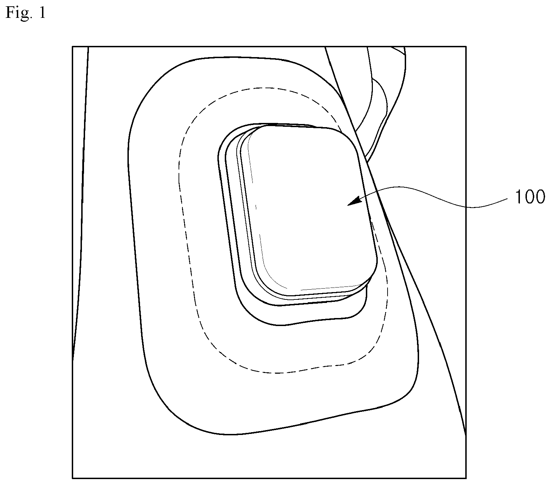

As an example, as shown in FIG. 1, the monitoring device 100 according to one embodiment of the present disclosure may be configured in the form of a patch that can be attached to a thigh portion of the subject.

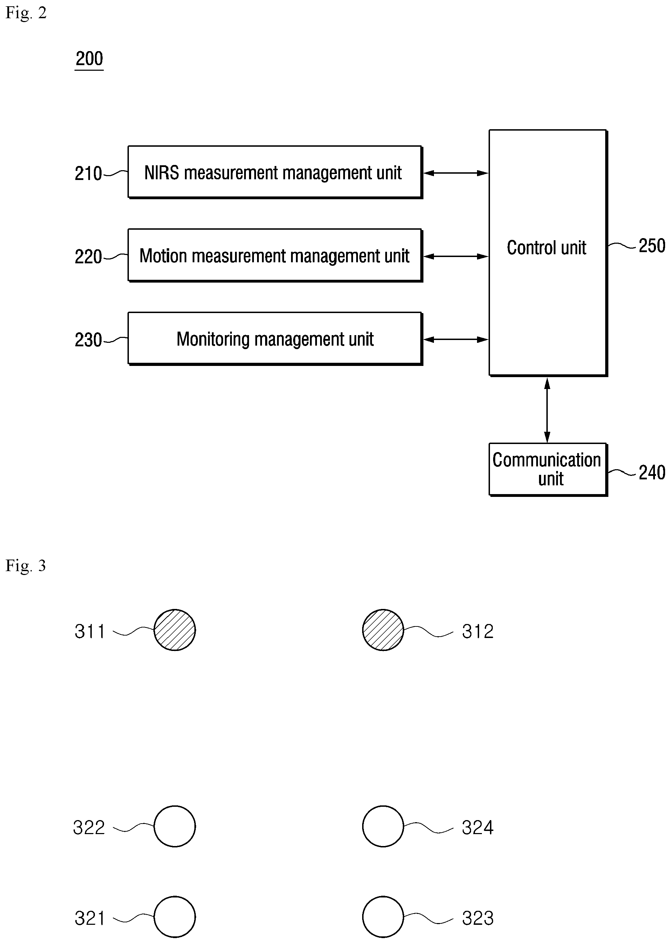

FIG. 2 illustratively shows an internal configuration of the monitoring system according to one embodiment of the present disclosure.

Referring to FIG. 2, the monitoring system 200 includes a near-infrared spectroscopy (NIRS) measurement management unit 210, a motion measurement management unit 220, a monitoring management unit 230, a communication unit 240, and a control unit 250. According to one embodiment of the present disclosure, at least some of the NIRS measurement management unit 210, the motion measurement management unit 220, the monitoring management unit 230, the communication unit 240, and the control unit 250 may be program modules to communicate with an external system (not shown). Such program modules may be included in the monitoring system 200 in the form of operating systems, application program modules, and other program modules, while they may be physically stored in a variety of commonly known storage devices. Further, the program modules may also be stored in a remote storage device that may communicate with the monitoring system 200. Meanwhile, the program modules may include, but not limited to, routines, subroutines, programs, objects, components, data structures, and the like for performing specific tasks or executing specific abstract data types as will be described below according to the present disclosure.

Although the monitoring system 200 is described as above, such a description is illustrative, and it will be apparent to those skilled in the art that at least a part of the components or functions of the monitoring system 200 may be implemented inside or included in the monitoring device as a wearable device which is to be worn (or attached) to the body portion of the subject, as necessary. Further, in some cases, all the functions and all the components of the monitoring system 200 may be implemented entirely inside the monitoring device 100 or may be included entirely in the monitoring device 100.

First, according to one embodiment of the present disclosure, the NIRS measurement management unit 210 may function to cause the plurality of light emitting units included in the monitoring device 100 to generate optical signals toward a body tissue located in a body portion of the subject. Specifically, according to one embodiment of the present disclosure, the NIRS measurement management unit 210 may cause the plurality of the light emitting units to generate an optical signal independently of each other in accordance with a time division manner or a frequency division manner.

Further, according to one embodiment of the present disclosure, the NIRS measurement management unit 210 may function to cause the plurality of light reception units included in the monitoring device 100 to detect the optical signals generated by the plurality of light emitting units with the distinction of the corresponding light emission units. The NIRS measurement management unit 210 according to one embodiment of the present disclosure may function to dynamically control measurement circuit gains of the plurality of light reception units in accordance with a time interval set based on a time division manner applied to the plurality of light emitting units, or modulate the optical signals detected by the plurality of light reception units based on a frequency division manner applied to the plurality of light emitting units, thereby allowing the plurality of light reception units to detect the optical signals generated by the plurality of light emitting units with the distinction of the corresponding light emission units.

As an example, the NIRS measurement management unit 210 according to one embodiment of the present disclosure may function to, by referring to a distance between an m-th light emitting unit among the plurality of light emitting units and an n-th light reception unit among the plurality of light reception units, dynamically control the measurement circuit gain that the n-th light reception unit uses to detect the optical signal generated from the m-th light emitting unit.

Further, since measurement results may vary depending on a body portion (or a body tissue) the monitoring device 100 is attached, or a wireless or wired communication environment in which measurement is performed, the NIRS measurement management unit 210 according to one embodiment of the present disclosure may dynamically control the measurement circuit gain of the monitoring device 100 depending on a body portion to be monitored or an environment in which the biosignal is measured.

The NIRS measurement management unit 210 functions to determine, with reference to a position of an m-th light emitting unit among the plurality of light emitting units configured to generate optical signals toward the body tissue to be monitored, a position of an n-th light reception unit among the plurality of light reception units configured to detect the optical signals from the body tissue, and a distance between the m-th light emitting unit and the n-th light reception unit, an area (i.e., a subject area) and a depth (i.e., a subject depth) of the body tissue which corresponds to the pair of the m-th light emitting unit and the n-th light reception unit. According to one embodiment of the present disclosure, the greater the distance between the m-th light emitting unit and the n-th light reception unit, the greater the depth of the body tissue that can be measured based on the optical signals generated by the m-th light emitting unit and detected by the n-th light reception unit.

The NIRS measurement management unit 210 functions to measure hemodynamics of the body tissue at the area and the depth corresponding to the pair of m-th light emitting unit and n-th light reception unit based on the optical signal generated by the m-th light emitting unit and detected by the n-th light reception unit.

The NIRS measurement management unit 210 functions to estimate the activity of the body tissue for each area and each depth based on the hemodynamics in at least one area and at least one depth of the body tissue.

The motion measurement management unit 220 functions to estimate the exercise quantity of the subject based on information on posture or motion of the body portion associated with the body tissue to be monitored. For example, when the body tissue to be monitored is a thigh muscle, the motion measurement management unit 220 according to one embodiment of the present disclosure estimates the exercise quantity of the subject based on information on the posture or motion of a leg portion.

As an example, the motion measurement management unit 220 according to one embodiment of the present disclosure may calculate velocities in the x-axis direction, the y-axis direction, and the z-axis direction by integrating an acceleration value of a body portion measured by a certain sensing module, and estimate the exercise quantity of the subject based on the calculated velocity values. In this case, an acceleration direction or a movement direction of the monitoring device may vary depending on a type of exercise the subject is doing, or which body portion the subject attaches the monitoring device, and thus, according to one embodiment of the present disclosure, characteristic values to be applied to the information on the posture and motion of the monitoring device may be adaptively set in accordance with the type of exercise or the respective body tissue.

To this end, the monitoring device according to one embodiment of the present disclosure may include at least one technical means capable of acquiring physical information on the posture or motion of the monitoring device mounted to the body portion (e.g., leg part, or the like) of the subject. Examples of such technical means may include sensing modules such as a motion sensor, an acceleration sensor, a gyroscope, a magnetic sensor, a positioning module (a GPS module, a beacon-based positioning (confirming) module, or the like), a barometer, a distance sensor, a camera, and the like, which are commonly known.

According to one embodiment of the present disclosure, the monitoring management unit 230 may function to provide various monitoring information related to the activity of the body tissue of the subject based on the information measured (or estimated) by the NIRS measurement management unit 210 or the motion measurement management unit 220.

The monitoring management unit 230 functions to evaluate the exercise efficiency of the body tissue of the subject and provide information on the evaluation result by referring to the information on the hemodynamics measured by the NIRS measurement management unit 210 and the information on the exercise quantity estimated by the motion measurement management unit 220.

Specifically, the monitoring management unit 230 according to one embodiment of the present disclosure calculates the amount of oxygen consumption per unit exercise quantity (i.e., Mom/dTSI), and determines that as the calculated amount increases, the exercise efficiency (or exercise capacity) of the muscle of the subject is decreased. In an embodiment not according to the present invention it may be alternatively determined that the fatigue of the muscle is high. In addition, the monitoring management unit 230 according to one embodiment of the present disclosure may provide information on the degree of improvement in the exercise capacity of the subject and various recommendation information necessary for improving the exercise capacity of the subject by periodically performing the above-described evaluation.

The communication unit 240 according to one embodiment of the present disclosure may function to enable the monitoring system 200 to communicate with an external device.

The control unit 250 according to one embodiment of the present disclosure may function to control the flow of data among the NIRS measurement management unit 210, the motion measurement management unit 220, the monitoring management unit 230, and the communication unit 240. That is, the control unit 250 may control the flow of data from/to the outside of the monitoring system 200, or the flow of data among the respective components of the monitoring system 200, such that the NIRS measurement management unit 210, the motion measurement management unit 220, the monitoring management unit 230, and the communication unit 240 may carry out their particular functions, respectively.

### Exemplary embodiments

FIG. 3 illustratively shows an arrangement of the plurality of light emitting units and the plurality of light reception units according to one embodiment of the present disclosure.

According to one embodiment of the present disclosure, as shown in FIG. 3, two light emitting units 311 and 312 and four light reception units 321 to 324 included in the monitoring device may be arranged in a predetermined pattern. For example, a distance between the first light emitting unit 311 and the first light reception unit 321 may be set to 2.5 cm, a distance between the first light emitting unit 311 and the second light reception unit 322 may be set to 2 cm, a distance between the first light emitting unit 311 and the third light reception unit 323 may be set to 3.35 cm, and a distance between the first light emitting unit 311 and the fourth light reception unit 324 may be set to 3 cm. However, it should be understood that the arrangement of the light emitting units and the light reception units according to the present disclosure is not necessarily limited to the above example, but may be variously modified as long as it can achieve the objects of the present disclosure.

Further, according to one embodiment of the present disclosure, as shown in FIG. 3, the two light emitting units 311 and 312 may be configured to sequentially generate optical signals in a mutually exclusive manner in accordance with a time division manner. This makes it possible to prevent a near-far problem (an optical signal (i.e., an optical signal having a small signal intensity) originating from a relatively distant place may not be measured due to an optical signal (i.e., an optical signal having a large signal intensity) originating from a relatively near place), which may be caused when two or more light emitting units generate optical signals simultaneously.

Furthermore, according to one embodiment of the present disclosure, as shown in FIG. 3, the measurement circuit gain of the four light reception units 321 to 324 may be dynamically controlled based on a distance (e.g., 2.5 cm, 2 cm, 3.35 cm, 3 cm, or the like) from one of the light emitting units 311 and 312 as a target which generates the optical signal to be measured. For example, as the distance between the target light emitting unit and the light reception unit increases, the intensity of the optical signal measured by the light reception unit may be decreased, and thus, the measurement circuit gain of the light reception unit may be determined to be high. In contrast, for example, as the distance between the target light emitting unit and the light reception unit decreases, the intensity of the optical signal measured by the light reception unit may be increased, and thus, the measurement circuit gain of the light reception unit may be determined to be low.

According to one embodiment of the present disclosure, the NIRS measurement management unit 210 may function to manage a measurement channel, which is defined by a corresponding pair of light emitting unit and light reception unit among the plurality of light emitting units and the plurality of light reception units. According to one embodiment of the present disclosure, when an optical signal, which is generated by a specific light emitting unit disposed at a certain location inside the monitoring device, passes through (specifically, reflects or scatters at or transmits through) a body tissue of the subject, the optical signal may be detected by a specific light reception unit disposed at a certain location within the monitoring device. Thus, a channel (path or area) through which the optical signal is delivered (propagates or transmitted) may be defined as the measurement channel.

Specifically, the NIRS measurement management unit 210 according to one embodiment of the present disclosure is configured to define the measurement channel corresponding to the pair of the specific light emitting unit and the specific light reception unit which are located to have a predetermined distance therebetween. In addition, the NIRS measurement management unit 210 according to one embodiment of the present disclosure may be configured to manage a plurality of measurement channels defined to correspond to a plurality of pairs of light emitting units and light reception units which are paired as described above, in accordance with respective distances between the corresponding light emitting units and the corresponding light reception units (i.e., signal strengths of optical signals generated from the corresponding light emitting units and detected by the corresponding light reception units).

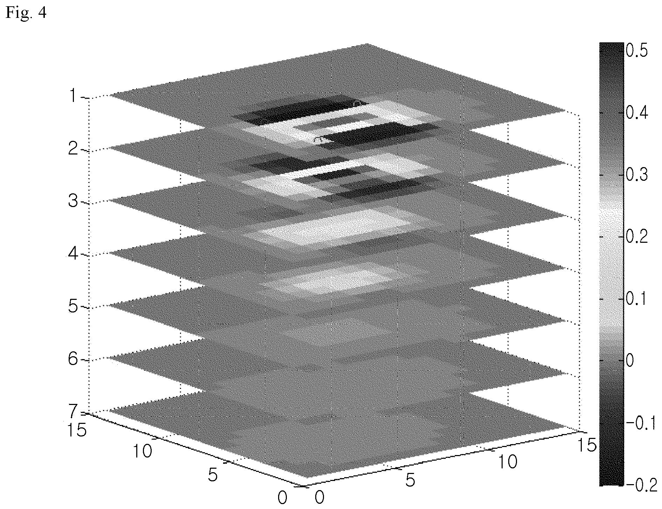

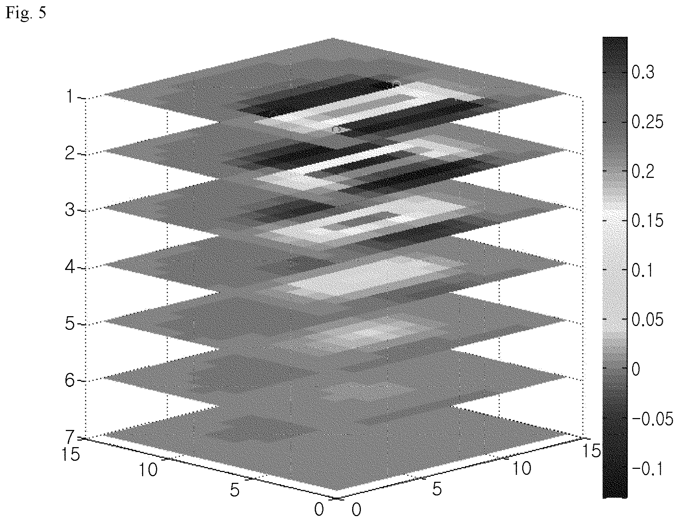

FIGS. 4 and 5 illustratively show how a depth to be measured of a body tissue varies with a distance between a light emitting unit and a light reception unit which are paired to define a measurement channel according to one embodiment of the present disclosure, respectively.

In the embodiment illustrated in FIGS. 4 and 5, the body tissue to be monitored was modeled in a three-dimensional space with a voxel having a size of 0.4 cm×0.4 cm×0.4 cm as a unit space. Further, in the embodiment illustrated in FIGS. 4 and 5, information on which voxel an optical signal generated from a specific light emitting unit and detected by a specific light reception unit passes in the three-dimensional space corresponding to the body tissue through, is visually expressed. When there is a specific voxel having a region of interest (ROI) of a high value in the three-dimensional space, it may mean that the optical signal is more likely to pass through the specific voxel (or the intensity of the optical signal passing through the specific voxel is large). Further, in the embodiment illustrated in FIGS. 4 and 5, the fact that the optical signal received by the light reception unit is more likely to pass through a specific voxel means that the optical signal is likely to be reflected or scattered at or transmitted through the specific voxel when passing therethrough, and thus, the optical signal may be regarded to have a large amount of information on the bioactivity occurring in the specific voxel.

Referring to FIG. 4, the optical signal received through the measurement channel in which the distance between the light emitting unit and the light reception unit is relatively short (e.g., the measurement channel defined by the pair of first light emitting unit 311 and second light reception unit 322 having a distance of 2 cm therebetween) has been confirmed to be more likely to pass only through shallow portions (e.g., a first layer and a second layer) of the body tissue.

Referring to FIG. 5, the optical signal received through the measurement channel in which the distance between the light emitting unit and the light reception unit is relatively long (e.g., the measurement channel defined by the pair of first light emitting unit 311 and second light reception unit 322 having a distance of 3 cm therebetween) has been confirmed to be more likely to pass through deep portions (e.g., a third layer) of the body tissue.

Therefore, according to one embodiment of the present disclosure, the NIRS measurement management unit 210 may determine that the depth of the body tissue, which can be measured by the optical signal generated from the m-th light emitting unit and received by the n-th light reception unit, becomes deeper, as the distance between the m-th light emitting unit and the n-th light reception unit increases.

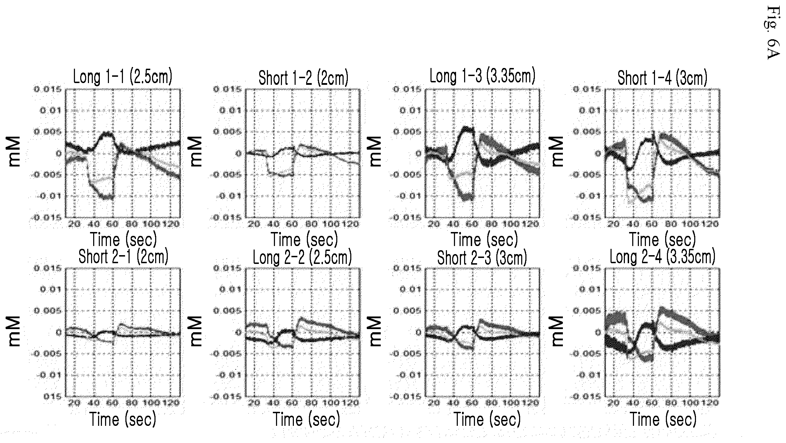

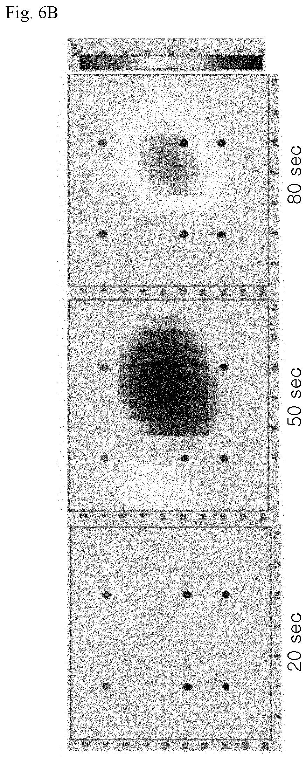

FIGS. 6A and 6B illustratively show a configuration in which the activity of a muscle being exercised is measured for each area based on an optical signal measured from the muscle being exercised according to one embodiment of the present disclosure.

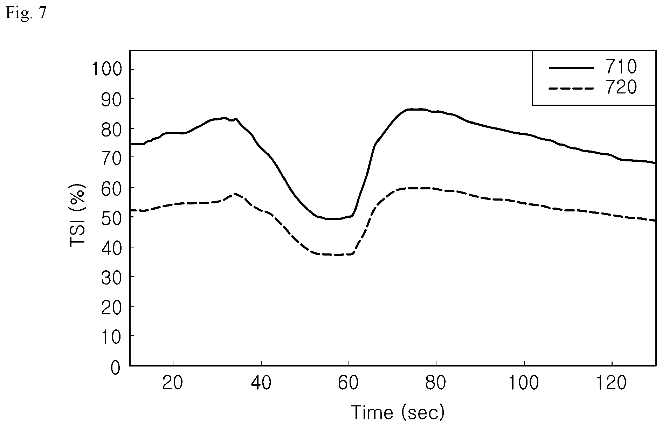

FIG. 7 illustratively shows a configuration in which the activity of the muscle being exercised is measured for each depth based on the optical signal measured from the muscle according to one embodiment of the present disclosure.

In the embodiment illustrated in FIGS. 6A, 6B, and 7, the subject performed an operation of contracting a muscle at a time point of 30 seconds, followed by an operation of relaxing the muscle at a time point of 60 seconds. An optical signal from the muscle of the subject was measured by using two light emitting units and four light reception units arranged as in the embodiment of FIG. 3.

Referring to FIG. 6A, the concentration of oxidized hemoglobin HbO2 and the concentration of non-oxidized hemoglobin HbR, which were measured through eight measurement channels defined by the combination of the two light emitting units and the four light reception units, are recorded as a function of time. Although the degree and aspect of change were different from each other depending on respective measurement channels, it can be seen that the amount of change in concentration between the oxidized hemoglobin and the non-oxidized hemoglobin was large in all the eight measurement channels at the time points of 30 seconds and 60 seconds (see eight graphs illustrated in FIGS. 6A).

Referring to FIG. 6B, at a time point of 20 seconds at which the subject does not perform a specific operation, the concentration of the oxidized hemoglobin in the entire area of the body tissue was confirmed to be uniform. At a time point of 50 seconds shortly after the muscle contraction operation, the concentration of the oxidized hemoglobin in a specific area of the body tissue in which oxygen was consumed was confirmed to be low. At a time point of about 80 seconds, which is a relatively long time after the muscle contraction operation, the concentration of the oxidized hemoglobin in the specific area of the body tissue in which the concentration of the oxidized hemoglobin was low was confirmed to be high.

Referring to FIG. 7, it can be seen that in both a relatively deep portion 710 and a relatively shallow portion 720 from the skin among the body tissues, the concentration of the oxidized hemoglobin was rapidly lowered after the time point of 30 seconds at which the muscle contraction operation is performed, and after several tens of times, went higher than the level prior to the muscle contraction operation and then returned to the level prior to the muscle contraction operation.

Although the case in which biological information measured by the NIRS measurement management unit 210 is the concentration of the oxidized hemoglobin or the concentration of the non-oxidized hemoglobin, has been mainly described in the above embodiments, the biological information that can be measured by the NIRS measurement management unit 210 is not necessarily limited thereto. It should be noted that various other types of biological information may be used as long as it can achieve the effects or objects of the method and the system therefor, and the non-transitory computer-readable recording medium described herein.

Further, although the case in which the body tissue to be monitored is a muscle has been mainly described in the above embodiments, the body tissue to be monitored according to the present disclosure is not necessarily limited thereto. It should be noted that various other body tissues may be objects to be monitored as long as they can be monitored based on hemodynamics.

According to one embodiment of the present disclosure, the body tissue to be monitored may be modeled in a heterogeneous space (i.e., a heterogeneous diffusion model) having a plurality of three-dimensional unit spaces (i.e., voxels) that may have different light absorption characteristics. Further, according to one embodiment of the present disclosure, the light irradiated from the light emitting unit of the monitoring device may be incident on all voxels constituting the body tissue included in the subject to be measured, and a light which transmits through a certain voxel or a light which is reflected from the certain voxel and detected by the light reception unit may include information on the certain voxel. Therefore, according to one embodiment of the present disclosure, the optical signal detected by the light reception unit may be configured as a sum of a plurality of unit optical signals reflecting the influence (or contribution) from each of the plurality of voxels. Further, the optical signal detected by the light reception unit may be used to find out the light absorption characteristics of the body tissue to be measured for each voxel (or each depth) by using a Diffuse Optical Tomography (DOT) technique which reconstructs the light absorption characteristics of each of the plurality of voxels constituting a body tissue (defined as a heterogeneous diffusion model) to be measured from a plurality of actual measurement signal values measured by each of the plurality of light reception units included in the monitoring device.

To improve the reliability of the diffusion optical tomography technique, it requires measurement channels defined by a plurality of light emitting units and a light reception unit. According to the present disclosure, measurement channels which are defined by a plurality of light emitting units and a plurality of light reception units arranged at predetermined intervals and has various combinations of distances between the respective light emitting units and the respective light reception units, can be implemented. With this configuration, it is possible to provide beneficial effects of improving accuracy and reliability in estimating (or calculating) the light absorption characteristics for each voxel (or each depth) by using the diffusion optical tomography technique.

The embodiments according to the present disclosure as described above may be implemented in the form of program instructions that can be executed by various computer components, and may be stored on a non-transitory computer-readable recording medium. The non-transitory computer-readable recording medium may include program instructions, data files, and data structures and the like, separately or in combination. The program instructions stored on the non-transitory computer-readable recording medium may be specially designed and configured for the present disclosure, or may also be known and available to those skilled in the computer software field. Examples of the non-transitory computer-readable recording medium include the following: magnetic media such as hard disks, floppy disks and magnetic tapes; optical media such as compact disk-read only memory (CD-ROM) and digital versatile disks (DVDs); magneto-optical media such as floptical disks; and hardware devices such as read-only memory (ROM), random access memory (RAM) and flash memory, which are specially configured to store and execute program instructions. Examples of the program instructions include not only machine language codes created by a compiler, but also high-level language codes that can be executed by a computer using an interpreter or the like. The above hardware devices may be configured to operate as operate one or more software modules to perform the processes of the present disclosure, and vice versa.

## Claims

1. A method of monitoring a body tissue, comprising the steps of:
determining, with reference to a position of an m-th light emitting unit among a plurality of light emitting units (311, 312) configured to generate optical signals toward the body tissue to be monitored, a position of an n-th light reception unit among a plurality of light reception units (321, 322, 323, 324) configured to detect the optical signals from the body tissue, and a distance between the m-th light emitting unit and the n-th light reception unit, an area and a depth of the body tissue which corresponds to a pair of the m-th light emitting unit and the n-th light reception unit;
measuring hemodynamics of the body tissue at the area and the depth corresponding to the pair of the m-th light emitting unit and the n-th light reception unit based on the optical signal generated by the m-th light emitting unit and detected by the n-th light reception unit;
wherein the method further comprises the step of estimating an exercise quantity of a subject to be measured based on information on a posture or motion of a body portion associated with the body tissue;
estimating an activity of the body tissue on an area basis and a depth basis based on the hemodynamics at at least one of the area and the depth of the body tissue;
evaluating an exercise efficiency of the body tissue with reference to information on the measured hemodynamics and the information on the estimated exercise quantity,
wherein the body tissue is a muscle, and
wherein in the evaluation step, an amount of oxygen consumption per unit exercise quantity is calculated, and it is determined that as the calculated amount increases, the exercise efficiency of the muscle is decreased.

2. The method of Claim 1, wherein the optical signals generated by the plurality of light emitting units (311, 312) comprise near-infrared signals.

3. The method of Claim 1, wherein a measurement channel is defined to correspond to a pair of a specific light emitting unit among the plurality of light emitting units (311, 312) and a specific light reception unit among the plurality of light reception units (321, 322, 323, 324), which exist within a predetermined distance from each other.

4. The method of Claim 1, wherein as the distance between the m-th light emitting unit and the n-th light reception unit increases, the depth of a portion in the body tissue where the hemodynamics can be measured based on the optical signal generated by the m-th light emitting unit and detected by the n-th light reception unit becomes deeper.

5. The method of Claim 1, wherein the hemodynamics comprises at least one of a concentration of an oxidized hemoglobin, a concentration of a non-oxidized hemoglobin, an oxygen saturation, and the variation amount of blood flow.

6. A system for monitoring a body tissue, comprising:
a near-infrared spectroscopy (NIRS) measurement management unit (210) configured to determine, with reference to a position of an m-th light emitting unit among a plurality of light emitting units (311, 312) configured to generate optical signals toward the body tissue to be monitored, a position of an n-th light reception unit among a plurality of light reception units (321, 322, 323, 324) configured to detect the optical signals from the body tissue, and a distance between the m-th light emitting unit and the n-th light reception unit, an area and a depth of the body tissue which corresponds to the pair of the m-th light emitting unit and the n-th light reception unit,
measure hemodynamics of the body tissue at the area and the depth corresponding to the pair of the m-th light emitting unit and the n-th light reception unit based on the optical signal generated from the m-th light emitting unit and detected by the n-th light reception unit, and
estimate an activity of the body tissue on an area basis and a depth basis based on the hemodynamics at at least one of the area and the depth of the body tissue;
wherein the system further comprises a motion measurement management unit (220) configured to estimate an exercise quantity of a subject to be measured based on information on a posture or motion of a body portion associated with the body tissue; and
a monitoring management unit (230) configured to provide information on an activity of the body tissue on an area basis and a depth basis, to evaluate an exercise efficiency of the body tissue with reference to information on the measured hemodynamics and the information on the estimated exercise quantity,
wherein the body tissue is a muscle, and
wherein the monitoring management unit (230) is configured to calculate an amount of oxygen consumption per unit exercise quantity, and determine that as the calculated amount increases, the exercise efficiency of the muscle is decreased.

## Patentansprüche

1. Verfahren zum Überwachen eines Körpergewebes, umfassend die folgenden Schritte:
unter Bezugnahme auf eine Position einer m-ten Lichtemissionseinheit unter einer Vielzahl von Lichtemissionseinheiten (311, 312), die so konfiguriert sind, dass sie optische Signale in Richtung des zu überwachenden Körpergewebes erzeugen, Bestimmen einer Position einer n-ten Lichtempfangseinheit unter einer Vielzahl von Lichtempfangseinheiten (321, 322, 323, 324), die so konfiguriert sind, dass sie die optischen Signale von dem Körpergewebe erfassen, und eines Abstands zwischen der m-ten Lichtemissionseinheit und der n-ten Lichtempfangseinheit, eines Bereichs und einer Tiefe des Körpergewebes, entsprechend einem Paar der m-ten Lichtemissionseinheit und der n-ten Lichtempfangseinheit;
Messen der Hämodynamik des Körpergewebes in dem Bereich und der Tiefe entsprechend dem Paar der m-ten Lichtemissionseinheit und der n-ten Lichtempfangseinheit, basierend auf dem optischen Signal, das von der m-ten Lichtemissionseinheit erzeugt und von der n-ten Lichtempfangseinheit erfasst wird;
wobei das Verfahren ferner die folgenden Schritte umfasst:
Schätzen einer Übungsgröße eines zu messenden Subjekts basierend auf Informationen über eine Haltung oder Bewegung eines Körperbereichs, der dem Körpergewebe zugeordnet ist;
Schätzen einer Aktivität des Körpergewebes auf einer Flächenbasis und einer Tiefenbasis basierend auf der Hämodynamik bei mindestens einer der Fläche oder der Tiefe des Körpergewebes;
Bewerten einer Übungseffizienz des Körpergewebes unter Bezugnahme auf Informationen über die gemessene Hämodynamik und die Informationen über die geschätzte Übungsgröße,
wobei das Körpergewebe ein Muskel ist, und
wobei in dem Auswertungsschritt eine Menge des Sauerstoffverbrauchs pro Einheitsübungsgröße berechnet wird und bestimmt wird, dass die Übungseffizienz des Muskels abnimmt, wenn die berechnete Menge zunimmt.

2. Verfahren gemäß Anspruch 1, wobei die von der Vielzahl von Lichtemissionseinheiten (311, 312) erzeugten optischen Signale Signale im nahen Infrarotbereich umfassen.

3. Verfahren gemäß Anspruch 1, wobei ein Messkanal so definiert ist, dass er einem Paar aus einer bestimmten Lichtemissionseinheit unter der Vielzahl von Lichtemissionseinheiten (311, 312) und einer bestimmten Lichtempfangseinheit unter der Vielzahl von Lichtempfangseinheiten (321, 322, 323, 324) entspricht, die in einem vorbestimmten Abstand voneinander vorhanden sind.

4. Verfahren gemäß Anspruch 1, wobei mit zunehmendem Abstand zwischen der m-ten Lichtemissionseinheit und der n-ten Lichtempfangseinheit die Tiefe eines Bereichs in dem Körpergewebe, wo die Hämodynamik basierend auf dem durch die m-te Lichtemissionseinheit erzeugten optischen Signal gemessen und durch die n-te Lichtempfangseinheit erfasst werden kann, tiefer wird.

5. Verfahren gemäß Anspruch 1, wobei die Hämodynamik mindestens eines von einer Konzentration eines oxidierten Hämoglobins, einer Konzentration eines nicht oxidierten Hämoglobins, einer Sauerstoffsättigung und der Variationsmenge des Blutflusses umfasst.

6. System zum Überwachen eines Körpergewebes, umfassend:
eine Nahinfrarotspektroskopie (NIRS)-Messungsverwaltungseinheit (210), die so konfiguriert ist, dass sie, unter Bezugnahme auf eine Position einer m-ten Lichtemissionseinheit unter einer Vielzahl von Lichtemissionseinheiten (311, 312), die so konfiguriert sind, dass sie optische Signale in Richtung des zu überwachenden Körpergewebes erzeugen, eine Position einer n-ten Lichtempfangseinheit unter einer Vielzahl von Lichtempfangseinheiten (321, 322, 323, 324), die so konfiguriert sind, dass sie die optischen Signale von dem Körpergewebe erfassen, und einen Abstand zwischen der m-ten Lichtemissionseinheit und der n-ten Lichtempfangseinheit, einen Bereich und eine Tiefe des Körpergewebes entsprechend dem Paar der m-ten Lichtemissionseinheit und der n-ten Lichtempfangseinheit bestimmt,
die Hämodynamik des Körpergewebes in dem Bereich und der Tiefe misst, welches dem Paar aus der m-ten Lichtemissionseinheit und der n-ten Lichtempfangseinheit zugehörig ist, basierend auf dem optischen Signal, das von der m-ten Lichtemissionseinheit erzeugt und durch die n-te Lichtempfangseinheit erfasst wird, und
eine Aktivität des Körpergewebes auf einer Flächenbasis und einer Tiefenbasis basierend auf der Hämodynamik bei mindestens einem der Fläche oder der Tiefe des Körpergewebes schätzt;
wobei
das System ferner eine Bewegungsmessungs-Verwaltungseinheit (220) umfasst, die so konfiguriert ist, dass sie eine Übungsgröße eines zu messenden Subjekts basierend auf Informationen über eine Haltung oder Bewegung eines mit dem Körpergewebe verbundenen Körperbereichs schätzt; und
eine Überwachungsmanagementeinheit (230), die so konfiguriert ist, dass sie Informationen über eine Aktivität des Körpergewebes auf einer Flächenbasis und einer Tiefenbasis bereitstellt, um eine Übungseffizienz des Körpergewebes unter Bezugnahme auf Informationen über die gemessene Hämodynamik und die Informationen über die geschätzte Übungsgröße zu bewerten, und
wobei das Körpergewebe ein Muskel ist, und
wobei die Überwachungsmanagementeinheit (230) so konfiguriert ist, dass sie eine Menge an Sauerstoffverbrauch pro Einheitsübungsgröße berechnet und feststellt, dass die Übungseffizienz des Muskels abnimmt, wenn die berechnete Menge zunimmt.

## Revendications

1. Procédé de surveillance d'un tissu corporel, comprenant les étapes suivantes :
la détermination, en référence à une position d'une m-ième unité d'émission de lumière parmi une pluralité d'unités d'émission de lumière (311, 312) configurées pour générer des signaux optiques vers le tissu corporel à surveiller, d'une position d'une n-ième unité de réception de lumière parmi une pluralité d'unités de réception de lumière (321, 322, 323, 324) configurées pour détecter les signaux optiques provenant du tissu corporel, et d'une distance entre la m-ième unité d'émission de lumière et la n-ième unité de réception de lumière, d'une aire et d'une profondeur du tissu corporel correspondant à une paire de la m-ième unité d'émission de lumière et la n-ième unité de réception de lumière ;
la mesure d'une hémodynamique du tissu corporel à l'aire et à la profondeur correspondant à la paire de la m-ième unité d'émission de lumière et la n-ième unité de réception de lumière sur la base du signal optique généré par la m-ième unité d'émission de lumière et détecté par la n-ième unité de réception de lumière ;
dans lequel le procédé comprend en outre les étapes suivantes :
l'estimation d'une quantité d'exercice d'un sujet à mesurer sur la base d'informations relatives à une posture ou un mouvement d'une partie corporelle associée au tissu corporel ;
l'estimation d'une activité du tissu corporel sur une base d'aire et sur une base de profondeur en fonction de l'hémodynamique à au moins l'une de l'aire et de la profondeur du tissu corporel ;
l'évaluation d'une efficacité d'exercice du tissu corporel en référence à des informations relatives à l'hémodynamique mesurée et aux informations relatives à la quantité d'exercice estimée,
dans lequel le tissu corporel est un muscle, et
dans lequel, à l'étape d'évaluation, une quantité de consommation d'oxygène par quantité d'exercice unitaire est calculée, et il est déterminé que l'efficacité d'exercice du muscle diminue au fur et à mesure de l'augmentation de la quantité calculée.

2. Procédé selon la revendication 1, dans lequel les signaux optiques générés par la pluralité d'unités d'émission de lumière (311, 312) comprennent des signaux de proche infrarouge.

3. Procédé selon la revendication 1, dans lequel un canal de mesure est défini pour correspondre à une paire d'une unité d'émission de lumière spécifique parmi la pluralité d'unités d'émission de lumière (311, 312) et une unité de réception de lumière spécifique parmi la pluralité d'unités de réception de lumière (321, 322, 323, 324), qui existent à une distance prédéterminée l'une de l'autre.

4. Procédé selon la revendication 1, dans lequel, au fur et à mesure de l'augmentation de la distance entre la m-ième unité d'émission de lumière et la n-ième unité de réception de lumière, la profondeur d'une partie dans le tissu corporel où l'hémodynamique peut être mesurée sur la base du signal optique généré par la m-ième unité d'émission de lumière et détecté par la n-ième unité de réception de lumière augmente.

5. Procédé selon la revendication 1, dans lequel l'hémodynamique comprend au moins l'une parmi une concentration d'une hémoglobine oxydée, une concentration d'une hémoglobine non oxydée, une saturation en oxygène, et la quantité de variation de débit sanguin.

6. Système de surveillance d'un tissu corporel, comprenant :
une unité de gestion de mesure de spectroscopie de proche infrarouge (NIRS) (210) configurée pour déterminer, en référence à une position d'une m-ième unité d'émission de lumière parmi une pluralité d'unités d'émission de lumière (311, 312) configurées pour générer des signaux optiques vers le tissu corporel à surveiller, une position d'une n-ième unité de réception de lumière parmi une pluralité d'unités de réception de lumière (321, 322, 323, 324) configurées pour détecter les signaux optiques provenant du tissu corporel, et une distance entre la m-ième unité d'émission de lumière et la n-ième unité de réception de lumière, une aire et une profondeur du tissu corporel correspondant à la paire de la m-ième unité d'émission de lumière et la n-ième unité de réception de lumière,
mesurer une hémodynamique du tissu corporel à l'aire et à la profondeur correspondant à la paire de la m-ième unité d'émission de lumière et la n-ième unité de réception de lumière sur la base du signal optique généré par la m-ième unité d'émission de lumière et détecté par la n-ième unité de réception de lumière, et
estimer une activité du tissu corporel sur une base d'aire et sur une base de profondeur en fonction de l'hémodynamique à au moins l'une de l'aire et de la profondeur du tissu corporel ;
dans lequel le système comprend en outre une unité de gestion de mesure de mouvement (220) configurée pour estimer une quantité d'exercice d'un sujet à mesurer sur la base d'informations relatives à une posture ou un mouvement d'une partie corporelle associée au tissu corporel ; et
une unité de gestion de surveillance (230) configurée pour fournir des informations relatives à une activité du tissu corporel sur une base d'aire et sur une base de profondeur, pour évaluer une efficacité d'exercice du tissu corporel en référence à des informations relatives à l'hémodynamique mesurée et aux informations relatives à la quantité d'exercice estimée,
dans lequel le tissu corporel est un muscle, et
dans lequel l'unité de gestion de surveillance (230) est configurée pour calculer une quantité de consommation d'oxygène par quantité d'exercice unitaire, et déterminer que l'efficacité d'exercice du muscle diminue au fur et à mesure de l'augmentation de la quantité calculée.
